(19) Europäisches Patentamt
European Patent Office
Office européen des brevets

(11) Publication number: **0 314 350 B1**

(12) **EUROPEAN PATENT SPECIFICATION**

(45) Date of publication of patent specification: **06.10.93**

(51) Int. Cl.5: **C07D 265/22**, C09D 11/00, B41N 3/00, C07D 413/12

(21) Application number: **88309627.3**

(22) Date of filing: **14.10.88**

(54) **Fluorescent compounds and their use.**

(30) Priority: **20.10.87 GB 8724570**

(43) Date of publication of application:
**03.05.89 Bulletin 89/18**

(45) Publication of the grant of the patent:
**06.10.93 Bulletin 93/40**

(84) Designated Contracting States:
**BE CH DE ES FR GB IT LI NL SE**

(56) References cited:

**CHEMICAL ABSTRACTS, vol. 89, no. 26, 25th December 1978, page 523, abstract no. 223403x, Columbus, Ohio, US; M.V. LOSEVA et al.: "Electronic spectra of 2-(2-arysulfonylaminophenyl)-4H-3,1-benzoxazin-4-ones"**

**CHEMICAL ABSTRACTS, vol. 76, no. 7, 14th February 1972, page 267, abstract no. 33569q, Columbus, Ohio, US; M.V. LOSEVA et al.: "4H-3,1-Benzoxazin-4-ones. Methoxy-substituted 2-(2-arenesulfonylaminophenyl)-4H-3,1-benzoxazin-4-ones"**

(73) Proprietor: **THOMAS DE LA RUE & COMPANY LIMITED**
**6 Agar Street**
**London WC2N 4DE(GB)**

(72) Inventor: **Bratchley, Robin**
**30 Bingley Grove,**
**Woodley**
**Reading, Berkshire(GB)**
Inventor: **Cotterill, Phillip John**
**40 Plas Tirion Avenue,**
**Prestatyn**
**Clwyd, North Wales(GB)**
Inventor: **Haslop, John Martin**
**22 Radcott Close,**
**Woodley**
**Reading, Berkshire(GB)**
Inventor: **Smith, Peter Noel**
**18 Llewelyn Court,**
**Phyle**
**Clwyd, North Wales(GB)**
Inventor: **Tyson, Robert Graham**
**15 Aberconway Park,**
**Prestatyn**
**Clwyd, North Wales(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 314 350 B1

**CHEMICAL ABSTRACTS, vol. 77, no. 25, 18th December 1972, page 411, abstract no. 164616w, Columbus, Ohio, US; B.M. BOLOTIN et al.: "4H-3,1-Benzoxazin-4-one. IV. Interaction of arylsulfonylchlorides with anthranilic acid"**

(74) Representative: **Perry, Robert Edward et al**
**GILL JENNINGS & EVERY,**
**Broadgate House,**
**7 Eldon Street**
**London EC2M 7LH (GB)**

## Description

### Field of the Invention

This invention relates to fluorescent compounds and their use in inks and for security printing.

### Background of the Invention

Fluorescent compounds have hitherto been used in security printed documents such as banknotes, cheques, identification cards, credit cards and bank cards, to permit an unobvious form of authentication. Typically, a fluorescent compound is formulated into a printable composition which may contain other ingredients such as dyes or pigments. The composition is then printed on to the substrate, allowing the document to be authenticated by establishing the fluorescence occurring on irradiation at a known wavelength, e.g. 365 nm. Authentication may be made more specific by using fluorescent patterns or printed designs.

When used for authentication in a security document such as a banknote, it is extremely important that the fluorescent compound lasts for the lifetime of the document. This places certain unique and particularly stringent requirements on the properties of suitable fluorescent compounds.

Compounds with poor lightfastness are unacceptable, as are those which are not permanent, i.e. compounds which do not physically remain in place on the document. Nor should the compounds excessively diffuse when subjected to heat or dampness, or when placed in contact with other surfaces. Even slight diffusion might have an adverse effect on the clarity of a finely-printed fluorescent pattern. Another important factor is that the compound is preferably available for use at a purity of at least 98%.

Prior art relating to the fluorescent properties of compounds of formula I (see formulae, below) is largely the work of Russian authors/inventors. Many of their articles concentrate on the intra-molecular hydrogen bonding which apparently exists between the N atom in the oxazine ring and the H atom in the NH group. It is proposed that the strength of the hydrogen bond is influenced by substituents (e.g. $R^3 = NO_2$), with a consequent effect on the spectral and fluorescent characteristics of the compound as a whole.

GB-A-1070326 discloses, in particular, compounds I1 and I2 which each, when irradiated at 365 nm, fluoresce in the yellow to green part of the spectrum. This colour is often required, but the two compounds are far from ideal in another important test area. The naphthalene derivative (I2) has strong fluorescence intensity but poor light stability when used in a surface coating. Thus, after as little as 25 hours exposure to simulated daylight, the intensity is greatly reduced and, after 100 hours, almost non-existent. The p-toluene derivative (I1) has good light stability but a weaker fluorescence intensity, approximately 0.33 of that initially shown by I2. Poor fluorescence intensity means that an uneconomically large amount of the compound may have to be used to give the desired effect, and that in turn may limit the ability of an ink formulation to tolerate the addition of other pigments or dyes or other additives.

The synthetic process disclosed in GB-A-1070326 comprises condensing an optionally-substituted anthranilic acid (III) with 2 equivalents of the appropriate arylsulphonyl chloride ($Ar\text{-}SO_2Cl$) in the presence of pyridine. This procedure is not necessarily as straightforward as has been suggested, and difficulties may be encountered in obtaining the generally-desired purity level of 98% w/w or more. For example, a desired product may need to be purified from a level of, say, 70-90% to the desired minimum of 98%. Lengthy purification by, for example, column chromatography is necessary.

Chem. Abs. 89: 223403x (1978) disclose compounds of formula I including I3. Neither there nor in the corresponding paper by Loseva et al are any synthetic procedures given. It is concluded that the electron effect of a substituent ($NO_2$ or $OCH_3$) on the intramolecular hydrogen bond is greater by virtue of substitution at the position analogous to $R^3$ in formula I than by substitution at the position analogous to a 4-R substituent in formula I.

Loseva et al, Khimiya Geterotsiklicheskikh Soedinenij 8 (1971) 1028-1032, disclose, inter alia, the four compounds of formula I wherein one of $R^1$, $R^2$, $R^3$ and $R^4$ is $OCH_3$, the others are H, and Ar is 4-methylphenyl. UV, IR and luminescence spectra are reported. It is stated that the position of the methoxy group affects the strength of the intramolecular hydrogen bond. Strengthening of this bond is said to shift the luminescence maximum into the shortwave region and to increase luminescence intensity.

B. M. Bolotin, Chem Abs. 77: 164616w (1972), discloses 4H-3,1-benzoxazin-4-one compounds related to formula I, showing their synthesis. I3 is specified as an intermediate.

USSR Inventors' Certificates Nos. 279333 and 321129 describe the use of what is probably I1 as a luminescent agent respectively for postage stamp paper and for letterpress inks for postage stamps.

Loseva et al, Khimiya geterotsiklicheskikh soedinenig 1972 (No. 5) 616-621, describe substituted analogues of I1.

USSR Inventor's Certificate 323402 describes the synthesis of potentially luminescent esters from I1.

FR-A-2549055 describes 1,4-benzoxazin-2-ones and their use in changing the frequency of light and as lasers.

GB-A-1347455 describes various quinazolinones of formula II, their luminescence and their use in printing inks and for the purposes of identification and security. However, these compounds typically exhibit poor light stability. The corresponding benzoxazinones of formula I, in which $R^1$ or $R^2$ and $R^3$ or $R^4$ are each H, $C_{1-4}$ alkyl, $C_{1-4}$ alkoxy or halogen, and Ar is optionally-substituted phenyl or naphthyl (including I3) are disclosed as intermediates.

It is an object of this invention to provide fluorescent compounds having improved properties for use in security documents, including improved fluorescence, light-fastness and/or permanence.

## Summary of the Invention

For use in security documents and fluoresent inks, according to the invention, compounds (many of which are novel) are of formula I, wherein $R^1$, $R^2$, $R^3$ and $R^4$ are independently H, Z or Z-substituted phenyl; and Ar is phenyl or a heterocyclic radical optionally substituted by Z; in which each Z is selected from $C_{1-4}$ alkyl, OH, $C_{1-4}$ alkoxy, halogen, $NH_2$, $C_{1-4}$ alkylamino, di($C_{1-4}$alkyl)amino and phenyl; provided that $R^1$, $R^2$, $R^3$ and $R^4$ are not all H when Ar is alkylphenyl or alkoxyphenyl. The heterocyclic radical may, for example, be derived from an aromatic ring having 4 or 5 C atoms and one or two O, S or N atoms, e.g. thiazole, thiophene or pyridine.

Novel compounds of formula I are defined in claim 11.

The invention relates also to processes for preparing such compounds and analogues thereof.

## Description of the Invention

Preferred compounds of formula I are those wherein $R^1$, $R^2$, $R^3$ and $R^4$ are independently selected from H and Cl or another halogen. More preferably, one of $R^1$ and $R^2$ is H, and one of $R^3$ and $R^4$ is H. As an alternative, $R^1$, $R^2$, $R^3$ and $R^4$ may each be H. It is generally preferred that $R^1 = R^3$ and $R^2 = R^4$.

Ar is preferably Z-substituted phenyl other than alkylphenyl or nitrophenyl. More preferably, Ar is biphenyl and/or 4-(Z-substituted)phenyl. As an alternative, Ar may be 2 or 3-thienyl or 2,3 or 4-pyridyl.

Compounds according to the invention can be prepared by generally known processes, e.g. by a process analogous to that disclosed in GB-A-1070326. More particularly, the novel compounds may be prepared by condensing an optionally-substituted anthranilic acid (III) with an arylsulphonyl chloride, preferably in an organic solvent such as pyridine.

This one-step method may give only moderate yields, and the crude product may contain between 15 and 50% w/w of an as yet unidentified impurity. Lengthy purification is necessary, and reduced yields must be expected. It has been observed that these disadvantages are somewhat reduced if $R^1$ or $R^2$ is Cl, i.e. the starting material is 4- or 5-chloroanthranilic acid, or perhaps another halogen. The method can then be used to produce a product more than 97% pure, and recrystallation from ethyl acetate can be sufficient to raise the purity to more than 99%. Ar may be any carbocyclic or heterocyclic aryl radical.

The one-step method produces compounds of formula I in which $R^1 = R^3$ and $R^2 = R^4$. A preferred method for preparing such compounds in general, i.e. in which Ar is not restricted to the definition given above but may be any substituent, including naphthyl or another fused aromatic ring, and in which $R^1$ and $R^2$ are not necessarily limited to the groups given above, comprises two steps. The first step involves reaction of an anthranilic acid (III) with thionyl chloride or another cyclising agent, to give an intermediate of formula IV. In the second step, this intermediate is reacted with the appropriate arylsulphonyl chloride (Ar-$SO_2$Cl) in which the aryl group may be carbocyclic or heterocyclic.

Owing to the better utilisation of the arylsulphonyl chloride in the two-step method by comparison with the one-step method, the former is particularly preferred when the arylsulphonyl chloride is a relatively expensive reagent. Recrystallisation, rather than column chromatography, may be sufficient to achieve the desired level of purity if the novel two-step synthesis is used.

Compounds of the invention are of value for printing security documents, banknotes or other substrates which may need to be "identified" by virtue of their ability to fluoresce on irradiation. For this purpose, compounds of the invention are suitably formulated into inks of a nature conventional for printing purposes.

Illustrative but non-limiting examples of security documents are banknotes, travellers' cheques, cheques, passports, identity cards, credit cards, cash cards, charge cards, security labels, security tickets,

e.g. airline and ferry tickets, bonds, tax stamps, licences, securities and certificates. Substrates are typically paper such as security grade paper, banknote paper, photographic grade paper, resin-coated paper or security grade card or plastics paper or plastics film or sheet. Typical plastics are polyester, polyvinyl choride, polyethylene and polypropylene.

For certain documents such as credit cards, identity cards and passports, the fluorescent compound may be covered by a protective layer of polymer or plastics film. This film may be heat-sealed and/or bonded with adhesive.

The fluorescent compounds will normally be incorporated into an ink formulation, e.g. offset lithographic (wet or dry), letterpress, flexographic, intaglio, screen or gravure or ink jet. Alternatively, the compounds may be applied in a paint or lacquer.

Illustrative ink formulations are as follows. Amounts are given in parts by weight.

| | |
|---|---|
| Offset Lithography: | |
| Fluorescent compound | 1-20 |
| Modified alkyd varnish mixture | 53-86 |
| Wax paste | 0-5 |
| Precipitated calcium carbonate | 9-40 |
| Solvent | 0-2 |
| Mixed driers | 0.8-1.5 |
| Letterpress: | |
| Fluorescent compound | 1-30 |
| Modified alkyd varnish mixture | 59-89 |
| Silicate extender | 5-13 |
| Mixed driers | 0.8-2.1 |
| Intaglio: | |
| Fluorescent compound | 1-10 |
| Resin and phenolic modified alkyd varnish mixture | 36-43 |
| Wax paste | 4-6 |
| Calcium carbonate | 40-58 |
| Driers | 0.8-1.5 |

The above inks can be used to produce fluorescent features (under 365 nm irradiation) which are not clearly visible in daylight. Coloured pigments can also be added to the formulations to produce fluorescent inks which are coloured in visible light.

Other printing processes may be used with appropriately formulated inks. Examples include screen, gravure and flexographic inks.

Fluorescent materials can also be incorporated into plastics as a solid dispersion or solute and can produce fluorescent effects in block form or sheet form. Examples are polyethylene and PVC. The fluorescent material could also be laminated between two plastics films, e.g. of polyester, by incorporation into the adhesive.

Fluorescent compounds may also be incorporated into blocking foil composition or alternatively be added to the wax coating or carbon type papers to produce fluorescent effects by pressure transfer.

For use in printing on a security document, the fluorescent compound should desirably be prepared to a purity of at least 98% (here and throughout the specification, purity values are given on a w/w basis) and should usually be in particulate form. The average particle size is preferably no more than 4 µm, more preferably no more than 2 µm, e.g. 1-2 µm with a maximum particle size of 5 µm, and ideally less than 1 µm. When dispersions are used. Milling may be used to achieve such particle sizes.

For other security printing applications, the fluorescent compounds may be dissolved in an appropriate solvent.

In use, the compound must exhibit good fluoresence intensity and stability, in particular light stability. In some circumstances, it may be appropriate to use a compound which has particularly good performance under one criterion and an unexceptional performance under another, but compounds of the invention generally provide a balance of properties preferable to compounds which have been, and still are, used commercially.

As indicated above, compound I2 exhibits poor light-fastness. Such a compound is unsuitable for use in documents such as banknotes and identity cards which have a long projected lifetime and where exposure to light is inevitable.

General permanance of the image is also important. Diffusion results in the loss of the clarity of the fluorescent image and, in an extreme case, fluorescence may disappear completely. This process may be accelerated by contact with, say, polyvinyl chloride in a wallet.

Where documents are handled repeatedly, such as currency, resistance to sweat and sebum (a secretion from the sebaceous glands) is important. Additionally, such documents must have a resistance to a variety of other potential hazards. These include chance or deliberate mistreatment by a range of aqueous solutions or organic solvents. The known compounds I1, I2 and I3 lack sufficient resistance of this type.

Compounds suitable for security printing may also usefully be selected to exhibit a yellow-orange colour of fluorescence rather than the more normal yellow-green. This allows further security to be included in the document.

The intensity of fluorescence is also important. For example, compound I1 has a relatively low fluorescence intensity in surface coatings and printing.

The following Examples 1 to 10 illustrate how novel compounds of the invention (I4 to I13) may be prepared. Example 11 illustrates the novel process of the invention, for the preparation of a known compound (I2).

Example 1 (I4)

Biphenyl-4-sulphonyl chloride (221.0 g, 0.87 mol) was added over 1 hour to a stirred solution of anthranilic acid (60.0 g, 0.44 mol) in pyridine (240 ml). The temperature was maintained at 20-30 °C. The title compound (42.0 g, 42% yield, 94% w/w) was obtained as a yellow amorphous solid.

The crude product (29.0 g) was recrystallised twice from chloroform/methanol (500 ml/450 ml and 600 ml/450 ml) by dissolving the solid in hot chloroform, filtering and then adding the methanol. The solid was filtered, washed with chloroform/methanol (15 ml/10 ml) and dried in vacuo at 35 °C to give I4, i.e. 2-[2-(4-biphenylylsulphonylamino)phenyl]-4H-3,1-benzoxazin-4-one (12.1 g, 98.2% w/w by HPLC area %) as a pale-yellow crystalline solid.

m.p.: 221-222 °C

IR (Nujol mull): $\nu_{max}$ = 1770, 1590, 1550, 1350, 1150 and 760 $cm^{-1}$

Example 2 (I5)

Anthranilic acid (15.0 g, 0.11 mol) was treated with 4-chlorophenylsulphonyl chloride (46.2 g, 0.22 mol) in pyridine solution as reported for Example 1. The title compound (19.4 g, 85.5% yield, 84.7% w/w) was obtained as a white microcrystalline solid.

The crude product (19.4 g) was slurried in dichloromethane (150 ml) for 0.5 hour and filtered to give a purified sample of the title compound (13.2 g). This solid was further purified by column chromatography using a column of silica gel (160 g, Merck type 7734) packed in dichloromethane. The column was eluted with dichloromethane and 50 ml fractions were collected. Fractions containing the title compound were combined and evaporated to dryness to give 8.2 g of I5 at >98% w/w. The material was slurried in methanol (50 ml) for 0.25 hour. The solid was filtered, washed with methanol (10 ml) and acetone (10 ml) and dried in vacuo at 30 °C to give I5, i.e. 2-[2-(4-chlorophenylsulphonylamino)phenyl]-4H-3,1-benzoxazin-4-one (7.7 g, 99.2% w/w) as a white microcrystalline solid.

m.p. 222-223 °C

IR (Nujol mull): $\nu_{max}$ = 1765, 1595, 1560, 1150 and 600 $cm^{-1}$

Example 3 (I6)

2-Amino-5-chlorobenzoic acid (20.0 g, 0.12 mol) was treated with 4-methylphenylsulphonyl chloride (49.0 g, 0.25 mol) in pyridine solution as reported for Example 1. The title compound (20.1 g; 72.7% yield; 98.0% w/w) was obtained as a pale green solid.

The crude material (19.5 g) was purified by column chromatography (200 g, Merck type 15111) by elution with dichloromethane in a manner similar to that reported for I5. The combined fractions were evaporated to dryness and slurried in methanol (100 ml) for 0.25 hour. The solid was filtered and washed with methanol (10 ml) and acetone (2 x 10 ml). Drying gave I6, i.e. 2-[2-(4-methylphenylsulphonylamino)-5-chlorophenyl]-6-chloro-4H-3,1-benzoxazin-4-one (12.1 g, 98.0% w/w) as a very pale-green microcrystalline solid.

m.p.: 237-239 °C

IR (Nujol mull): $\nu_{max}$ = 1765, 1590, 1240, 1150 and 825 $cm^{-1}$

Example 4 (I7)

A solution of biphenyl-4-sulphonyl chloride (43.9 g, 0.174 mol) in pyridine (200 ml) was added dropwise, over one hour, to a stirred solution of 2-amino-5-chlorobenzoic acid (15.0 g, 0.087 mol) in pyridine (100 ml) at 20-30 °C. The mixture was stirred for a further 2.5 hours and then the precipitate was filtered off. The precipitate was washed on the filter with acetone (2 x 60 ml), 2M HCl (60 ml), water (until pH 7) and finally more acetone (2 x 60 ml). Drying in vacuo gave I7 (10.0 g, 43.9% yield, 97.74% w/w by HPLC analysis) as a pale-yellow microcrystalline solid.

The crude product (9.2 g) was dissolved in hot ethyl acetate (2000 ml) and filtered. The solution was cooled and the precipitate was isolated, by filtration. The precipitate was washed on the filter with ethyl acetate and then dried in vacuo to give I7, i.e. 2-[2-(4-biphenylylsulphonylamino)-5-chlorophenyl]-6-chloro-4H-3,1-benzoxazin-4-one (7.3 g, 79.3% recovery, 99.7% w/w by HPLC analysis) as a pale-yellow crystalline powder.

m.p. 260-261 °C

IR (KBr disc): $\nu_{max}$ = 1760, 1590, 1465, 1240, 1200 and 1160 $cm^{-1}$

Example 5 (I8)

A solution of p-toluenesulphonyl chloride (44.4 g, 0.23 mol) in pyridine (200 ml) was added dropwise, over 1 hour, to a stirred solution of 2-amino-4-chlorobenzoic acid (20.0 g, 0.12 mol) in pyridine (120 ml) at 20-30 °C. The mixture was stirred for a further 30 minutes before the precipitate was filtered off. The precipitate was washed with acetone (2 x 30 ml), 2M HCl (80 ml), water (until pH 7) and finally acetone again (2 x 80 ml). Drying in vacuo gave the title compound (19.1 g, 69.2% yield, 98,35% w/w by HPLC analysis) as a pale-green solid.

The impure material (19.0 g) was recrystallised from hot ethyl acetate (4500 ml) to give, after drying in vacuo, I8, i.e. 2-[2-(4-methylphenylsulphonylamino)-4-chlorophenyl]-7-chloro-4H-3,1-benzoxazin-4-one (14.9 g, 78.5% recovery, 99.93% w/w by HPLC analysis) as a white crystalline powder.

m.p. 254-255.5 °C

IR (KBr disc): $\nu_{max}$ = 1775, 1590, 1555, 1345, 1230 and 1160 $cm^{-1}$.

Example 6 (I9)

A solution of biphenyl-4-sulphonyl chloride (28.7 g, 0.114 mol) in pyridine (175 ml) was added dropwise, over 1 hour, to a stirred solution of 2-amino-4-chlorobenzoic acid (10.0 g, 0.56 mol) in pyridine (125 ml) at 20-30 °C. The mixture was stirred for a further 30 minutes and then the precipitated solid was filtered. The precipitate was washed with acetone (2 x 40 ml), 2M HCl (40 ml), water (until pH 7) and finally acetone again (2 x 40 ml). Drying in vacuo gave I9 (10.5 g, 71.9% yield, 99.55% w/w by HPLC analysis) as a pale-green solid.

The crude benzoxazinone (10.0 g) was recrystallised from hot ethyl acetate (5000 ml) to give, after drying in vacuo, I9, i.e. 2-[2-(4-biphenylylsulphonylamino)-4-chlorophenyl-7-chloro-4H,3,1-benzoxazin-4-one (6.7 g, 67.0% recovery, 99.98% w/w by HPLC analysis) as an off-white crystalline powder.

m.p. 277-278 °C

IR (KBr disc): $\nu_{max}$ = 1750, 1590, 1555, 1340, 1235 and 1160 $cm^{-1}$.

Example 7 (I10)

To a solution of 2-amino-5-chlorobenzoic acid (20.0 g, 0.12 mol) in pyridine (125 ml) was added a solution of 4-chlorophenylsulphonyl chloride (48.5 g, 0.23 mol) in pyridine (150 ml) at 20-30 °C. After stirring for 1 hour the precipitated solid was filtered and washed with acetone (2 x 80 ml), 2M HCl (80 ml), water (until pH 7) and finally acetone again (2 x 80 ml). Drying in vacuo gave I10, i.e. 2-[2-(4-chlorophenylsulphonylamino)-5-chlorophenyl]-6-chloro-4H-3,1-benzoxazin-4-one (14.8 g, 51.2% yield, 99.84% w/w by HPLC analysis) as a very pale-green crystalline powder.

m.p. 228-229 °C

IR (KBr disc): $\nu_{max}$ = 1770, 1595, 1465, 1335, 1240 and 1155 $cm^{-1}$.

Example 8 (I11)

To a solution of 2-amino-5-chlorobenzoic acid (20.0 g, 0.12 mol) in pyridine (125 ml) was added a solution of 4-methoxyphenylsulphonyl chloride (47.6 g, 0.23 mol) in pyridine (150 ml) at 20-30 °C. After stirring for 1 hour, the precipitated solid was filtered and washed with acetone (2 x 80 ml), 2M HCl (80 ml), water (to pH 7) and finally more acetone (2 x 80 ml). Drying in vacuo gave the title compound (13.0 g, 45.5% yield, 98.3% w/w by HPLC analysis) as a very pale-green solid.

The impure material (13.0 g) was recrystallised from hot ethyl acetate (2000 ml) to give after drying in vacuo, I11, i.e. 2-[2-(4-methoxyphenylsulphonylamino)-5-chlorophenyl]-6-chloro-4H-3,1-benzoxazin-4-one (9.9 g, 76.2% recovery, 99.5% w/w by HPLC analysis) as an off-white crystalline powder.

m.p. 206-207 °C

IR (KBr disc): $\nu_{max}$ = 1770, 1590, 1470, 1240, 1150 and 830 $cm^{-1}$.

Example 9 (I12)

Thiophene-2-sulphonyl chloride (50.9 g; 0.28 mol) was melted and added dropwise to a solution of anthranilic acid (19.0 g; 0.14 mol) in pyridine (100 ml) at 45-55 °C over a 1 hour period. The resulting mixture was then stirred at 50-60 °C for 1 hour and at 15 °C for a further 2 hours. At this point, acetone (50 ml) was added, and the reaction mixture was stirred for 1 hour. The precipitate was filtered, washed well with acetone (2 x 50 ml), 2M hydrochloric acid (50 ml), water (to pH 7) and finally acetone again (2 x 50 ml). Drying in vacuo gave the product (18.0 g) as an off-white amorphous solid. The material was purified by crystallisation from an ethyl acetate/dichloromethane mixture to give 2-[2-(2-thienylsulphonylamino)-phenyl]-4H-3,1-benzoxazin-4-one (14.6 g; 53.5% yield; HPLC analysis 98,8% w/w) as a white, microcrystalline powder.

m.p. 214-215 °C.

I.R. (KBr disc): $\nu_{max}$ = 1760, 1600, 1340, 1245, 1150, 1140 and 760 $cm^{-1}$.

Example 10 (I13)

A solution of pyridine-3-sulphonyl chloride hydrochloride (83.0 g; 0.31 mol) in pyridine (40 ml) was added dropwise to a solution of anthranilic acid (21.0 g; 0.153 mol) in pyridine (45 ml) at 45-55 °C over a 1.5 hour period. The resulting reaction mixture was stirred at 50-60 °C for a further 2 hours, and then for 18 hours at 15-20 °C. Acetone (45 ml) was added and the precipitate was isolated by filtration. The solid was washed well with acetone (2 x 50 ml), 2M hydrochloric acid (50 ml), water (to pH 7) and finally acetone again (2 x 50 ml). Drying in vacuo gave the product as a pale-yellow solid (11.8 g). The material was crystallised from an ethyl acetate/dichloromethane mixture to give 2-[2-(3-pyridylsulphonylamino)phenyl]-4H-3,1-benzoxazin-4-one (10.8 g; 37.0% yield; HPLC analysis 99.4% w/w) as a pale-yellow powder.

m.p. 221-222 °C.

I.R. (KBr disc): $\nu_{max}$ = 1760, 1600, 1245, 1155 and 760 $cm^{-1}$.

Example 11 (I2)

Thionyl chloride (325.0 g, 2.73 mol) was added dropwise over 2.5 hours to a stirred solution of anthranilic acid (250.0 g, 1.82 m) in pyridine (575.0 g, 7.28 mol) at 20-30 °C. The mixture was stirred for a further 30 minutes before adding dichloromethane (1 l) and water (1 l). After stirring for 30 minutes, the organic phase was separated, washed with water (1 l x 2) and dried ($MgSO_4$). The dichloromethane solution was then filtered through a bed of silica (350 g, Merck type 15111), and the silica was washed with

dichloromethane (250 ml). The combined filtrates were evaporated to dryness at 35°C to give 2-(2-aminophenyl)-4H-3,1-benzoxazin-4-one (94.2g, 87.4% w/w by HPLC area percent, 38.0% yield) as a yellow amorphous solid.

m.p. 137-144°C

IR (KBr disc): $\nu_{max}$ = 1730, 1580, 1535, 1460, 1225 and 1210 $cm^{-1}$.

Naphthalene-2-sulphonyl chloride (10.4 g, 0.046 mol) in pyridine (20 ml) solution was added over 1 hour to a stirred suspension of 2-(2-aminophenyl)-4H-3,1-benzoxazin-4-one (10.0 g, 85,5% w/w pure, 0.036 m) in pyridine (30 ml) at 20-30°C. The mixture was stirred for a further 1 hour before filtering and washing with acetone (20 ml x 2), 2M HCl (35 ml), water (until the filtrate is pH 7) and acetone (20 ml x 2). Drying in vacuo at 40°C gave the title compound (10.1 g, 96.9% w/w by HPLC area percent, 63.5% yield) as a beige solid.

The crude product was dissolved in dichloromethane (250 ml) and stirred with carbon (2.5 g) for 1 hour. After removal of the carbon by filtration, the solution was evaporated to about one-fifth volume, before adding ethyl acetate (50 ml). The mixture was further evaporated to about 75 ml. The precipitated solid was filtered, washed with ethyl acetate (10 ml) and dried to give 2-[2-(2-naphthalenesulphonylamino)phenyl]-4H-3,1-benzoxazin-4-one (6.75 g, 99.0% w/w by HPLC area percent, 43.4% yield) as a pale-yellow crystalline solid.

m.p. 195-196°C

IR (KBr disc): $\nu_{max}$ = 1755, 1595, 1335, 1240, 1150 and 650 $cm^{-1}$.

## Testing and Evaluation

IR spectra and melting points of the novel compounds are given above. None of the novel compounds exhibited any odour, except I10 which had a slight odour of pyridine.

Novel compounds (I4-I13) and also known compounds (I1, I2, I3, II1, II2 and II4) have been tested for various characteristics pertaining to their utility as fluorescent ink components.

The thirteen compounds I1 to I13 are insoluble in distilled water and in 5M HCl. I1, I3, I6, I10, I11 and I13 are soluble, I5, I8 and I12 are partially soluble, and I2, I4, I7 and I9 are insoluble, in 2% NaOH. Solubilities were assessed visually by immersion of 0.5 g of the raw compound in 10 ml of the solution for one day at room temperature and pressure.

Inks were prepared from each of the compounds under test. 10 g of each ink were prepared by incorporating 20% by weight of the compound into the modified alkyd drying oil-based ink containing 12.5% silicate (extender), 79.6% varnish, 5.8% wax and 2.1% driers. The ink was mulled for at least 250 revolution in five lots until the fineness of grind (measured after each 50 rev, using a Hegman Gauge) stopped falling.

0.4 ml of each ink was printed by dry offset lithography on to plain white mould-made paper of low fluorescence, using 50 kgf on the IGT printability tester. The prints were left to dry at room temperature for four days before testing.

## Print Testing

Emission spectra and fluorescence intensities were recorded on a Perkin-Elmer luminescence spectrometer at an excitation wavelength of 364 nm. The results are tabulated below. In the table, "Fluorescence Intensity" means the relative fluorescence intensity of the print at the wavelength of maximum absorbance. "Loss in Int." means the instrumentally-measured loss in fluorescence intensity after 100 hours, at the wavelength of maximum absorbance.

In order to assess permanence of the compounds, 30 mm length IGT prints of each compound were tested for resistance to various environments and particular materials. The tested prints were assessed for loss of fluoresence by comparison with their reference (untested) prints, on a scale of 0 to 5. 5 indicates the best result, i.e. no loss in fluorescence; 4 indicates slight loss, 3 significant loss, 2 marked loss, 1 almost complete loss and 0 complete loss, in fluoresence.

The assessment values for resistance to light (lightfastness) at 25 and 100 hours (a particularly important criterion) in simulated daylight in accelerated tests (425 $J/cm^2/h$, 65% relative humidity) are tabulated below, as is the sum of assessed resistances to a sum of seven different environments. The ratings in parenthesis are respectively for resistance to:

dry heat (one week)
contact with plasticised PVC under pressure
immersion in industrial methylated spirits

immersion in artifical sebum
immersion in castor oil
immersion in white spirits
immersion in turpentine.

A score of 5 means excellent resistance, 3 good, and 0 extremely poor.

Immersion of the unprinted material in alkali solution was also assessed. A score of 5 means excellent permanence, and that the compound is effectively insoluble. A score of 0 means negligible permanence as visually assessed by residual fluorescence. The sum totals give a broad indication of the degree of permanence of the compounds.

A value of 5 was observed for all compounds (I1-I11) in tests for resistance to humidity (one week), acid sweat, alkali sweat (except that I2 and I4 gave 4), dry ironing (except that I10, I12 and I13 gave 4), wet ironing (except that I10 gave 4), bleach (except I13 gave 4), hot water (except I12 gave 3 and I13 gave 0), 2% alkali (except I13 gave 2), acid, aqueous sodium sulphide (except I12 gave 3 and I13 gave 0) and (40/60) petroleum ether. Zero resistance was recorded for all compounds under test with respect to acetone, ethyl acetate and xylene, except that I7, I8 and I9 each scored 1 against ethyl acetate and xylene, I4 scored 1 against xylene, I12 scored 1 against acetone and ethyl acetate and I13 scored 1 against acetone, ethyl acetate and xylene.

A subjective assessment of the colour of fluorescence on irradiation at 364 nm is given in the Table, as is the wavelength of maximum emission. The tabulated colours are abbreviated as G (green), Y (yellow), YG (yellow-green), YO (Yellow-orange), O (orange) and B (blue).

| Compound | Visually-assessed Resistance to Light (25hr) | (100hr) | Permanence (Spectrum of conditions) | Resistance to alkali | Fluorescence Intensity | Loss in Int. (%) at $\lambda_{max}$ | Wavelength of Max.Emission (nm) | Fluorescence Colour(approx) |
|---|---|---|---|---|---|---|---|---|
| I1 | 4 | 3 | 8(2311100) | 0 | 5 | 60 | 538 | YG |
| I2 | 2 | 1 | 12(3212211) | 5 | 15.5 | 80 | 523 | G |
| I3 | 4 | 3 | 12(2312211) | 0 | 11 | 50 | 531 | G-YG |
| I4 | 4 | 3 | 25(4454422) | 5 | 10 | 35 | 550 | O-YO |
| I5 | 4 | 3 | 8(2311100) | 3 | 9 | 37 | 532 | G-YG |
| I6 | 4 | 3 | 15(3352200) | 0 | 9 | 29 | 547 | YO-Y |
| I7 | 4 | 2 | 20(3353411) | 5 | 9 | 39 | 553 | O-YO |
| I8 | 4 | 2 | 19(3353311) | 3 | 8 | 47 | 534 | YG |
| I9 | 3 | 1 | 27(4454433) | 5 | 15.5 | 65 | 523 | G |
| I10 | 4 | 3 | 7(2230000) | 0 | 14.5 | 32 | 537 | Y-YG |
| I11 | 4 | 3 | 12(3251100) | 0 | 7 | 14 | 552 | O-YO |
| I12 | 4 | 3 | 16(5522200) | 3 | 11.4 | 41 | 534 | YG |
| I13 | 4 | 3 | 16(5422210) | 0 | 10.3 | 28 | 534 | YG |
| II1 | 3 | 2 | 22(5513350) | 0 | 15.8 | 68 | 528 | G |
| II2 | 1 | 0 | 13(4301140) | 3 | 9.4 | 100 | 523 | G |
| II4 | 2 | 0 | 27(5535450) | 5 | 15.7 | 98 | 529 | G |

The tabulated results show that I2, II2 and II4 have unacceptably poor, and II1 has poor, lightfastness. If, as is reasonable, 65% loss in intensity is accepted for utility (although 50% or lower is more reasonable), the only known compounds which meet this criterion (I1 and I3) are still poor and have low permanence.

Alkali resistance is preferably high, in order to be wash-tolerant, but in certain circumstances alkali-solubility can be tolerated. A reasonable combination of parameters for utility is a permanence value of at

least 8, a fluorescence intensity of at least 7 and an intensity loss of no more than 65% provided that, if two of the values are approached, the other is correspondingly good (as for I9, I5 inter alia). I4, I6, I7 and I11 have a desirable YO colour.

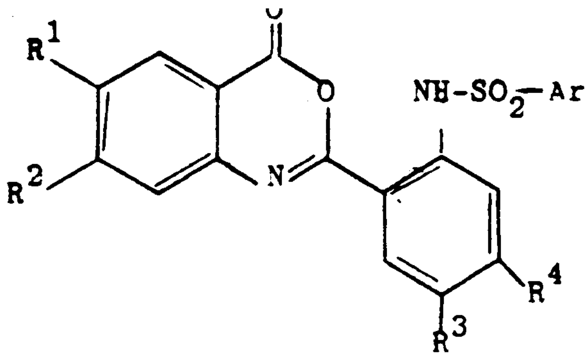

(I)

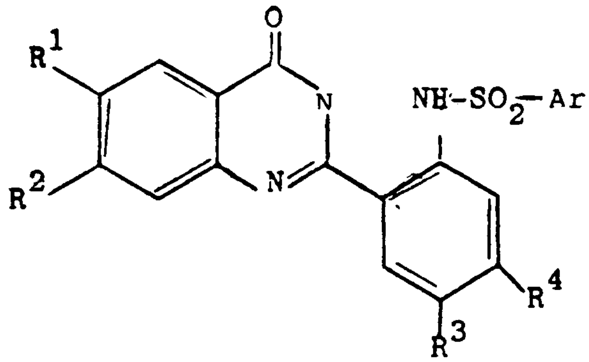

(II)

| | $R^1$ | $R^2$ | $R^3$ | $R^4$ | Ar |
|---|---|---|---|---|---|
| I1/II1 | H | H | H | H | 4-methylphenyl |
| I2/II2 | H | H | H | H | 2-naphthyl |
| I3 | H | H | H | H | 4-methoxyphenyl |
| I4/II4 | H | H | H | H | 4-biphenylyl |
| I5 | H | H | H | H | 4-chlorophenyl |
| I6 | Cl | H | Cl | H | 4-methylphenyl |
| I7 | Cl | H | Cl | H | 4-biphenylyl |
| I8 | H | Cl | H | Cl | 4-methylphenyl |
| I9 | H | Cl | H | Cl | 4-biphenylyl |
| I10 | Cl | H | Cl | H | 4-chlorophenyl |
| I11 | Cl | H | Cl | H | 4-methoxyphenyl |
| I12 | H | H | H | H | 2-thienyl |
| I13 | H | H | H | H | 3-pyridyl |

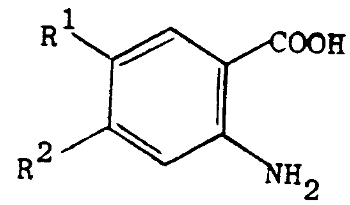

(III)

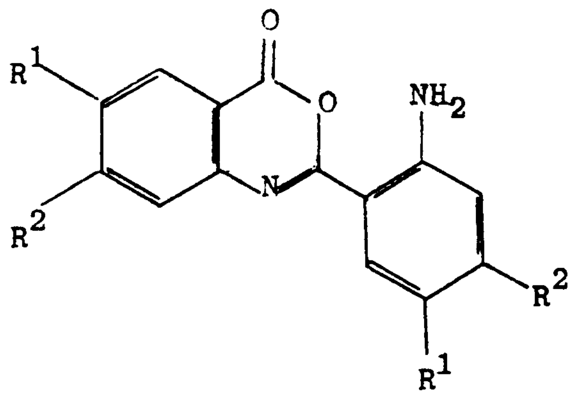

(IV)

## Claims

1. A fluorescent ink comprising a compound of formula I

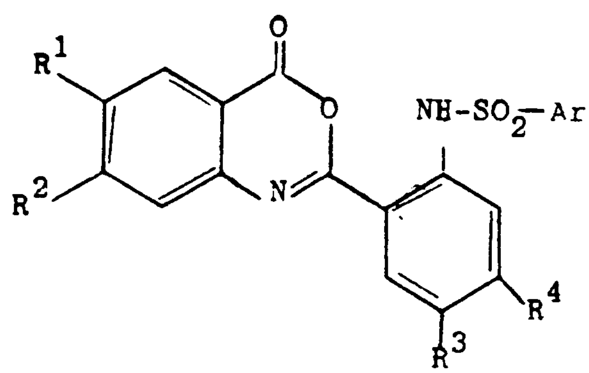

(I)

wherein $R^1$, $R^2$, $R^3$ and $R^4$ are independently H, Z or Z-substituted phenyl; and Ar is optionally Z-substituted phenyl or heterocyclyl; in which the Z's are independently selected from $C_{1-4}$ alkyl, OH, $C_{1-4}$ alkoxy, halogen, $NO_2$, $NH_2$, $C_{1-4}$ alkylamino, di($C_{1-4}$ alkyl)amino and phenyl; provided that $R^1$, $R^2$, $R^3$ and $R^4$ are not all H when Ar is alkylphenyl or alkoxyphenyl.

2. An ink according to claim 1, wherein $R^1$, $R^2$, $R^3$ and $R^4$ are independently selected from H and Cl or another halogen.

3. An ink according to either preceding claim, wherein $R^1 = R^3$ and $R^2 = R^4$.

4. An ink according to any preceding claim, wherein one of $R^1$ and $R^2$ is H, and one of $R^3$ and $R^4$ is H.

**5.** An ink according to claim 1, wherein $R^1$, $R^2$, $R^3$ and $R^4$ are each H.

**6.** An ink according to any preceding claim, wherein Ar is Z-substituted phenyl other than alkylphenyl or nitrophenyl.

**7.** An ink according to claim 6, wherein Ar is biphenylyl.

**8.** An ink according to any preceding claim, wherein Ar is 4-(Z-substituted)phenyl.

**9.** An ink according to any of claims 1 to 5, wherein Ar is 2 or 3-thienyl or 2,3 or 4-pyridyl.

**10.** A security document carrying a compound according to any preceding claim.

**11.** A compound of formula I as defined in any of claims 1 to 10, provided that, when $R^1$, $R^2$, $R^3$ and $R^4$ are selected from H, alkyl, alkoxy and halogen, Ar is not phenyl, alkylphenyl or alkoxyphenyl, and that the compound is not 2-[2-(4-nitrophenylsulphonylamino)phenyl]-4H-3,1-benzoxazin-4-one or 2-[2-(4-methylphenylsulphonylamino)-4-nitrophenyl]-4H-3,1-benzoxazin-4-one.

**12.** A compound of claim 11, which is 2-[2-(4-biphenylylsulphonylamino)phenyl]-4H-3,1-benzoxazin-4-one.

**13.** A compound of claim 11, which is 2-[2-(4-chlorophenylsulphonylamino)phenyl]-4H-3,1-benzoxazin-4-one.

**14.** A compound of claim 11, which is 2-[2-(4-methylphenylsulphonylamino)-5-chlorophenyl]-6-chloro-4H-3,1-benzoxazin-4-one.

**15.** A compound of claim 11, which is 2-[2-(4-chlorophenylsulphonylamino)-5-chlorophenyl]-6-chloro-4H-3,1-benzoxazin-4-one.

**16.** A process for preparing a compound of the formula shown in claim 1, wherein $R^1$ and $R^3$ or $R^2$ and $R^4$ are halogen, which comprises reacting a corresponding optionally-substituted anthranilic acid of formula III

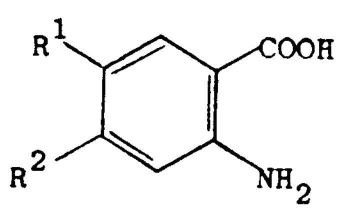

(III)

with a carbocyclic or heterocyclic arylsulphonyl chloride.

**17.** A process for preparing a compound of the formula shown in claim 1, $R^1$, $R^2$, $R^3$, $R^4$ and Ar optionally being as defined in claim 1, which comprises (1) reacting an anthranilic acid of formula III

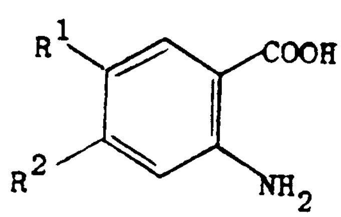

(III)

with $SOCl_2$; and (2) reacting the resultant intermediate of formula IV

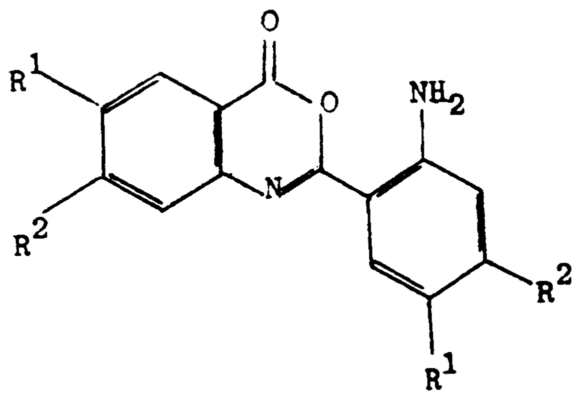

(IV)

with a carbocyclic or heterocyclic arylsulphonyl chloride of the formula Ar-$SO_2$Cl.

**Patentansprüche**

1. Fluoreszierende Farbe, die eine Verbindung der Formel I aufweist

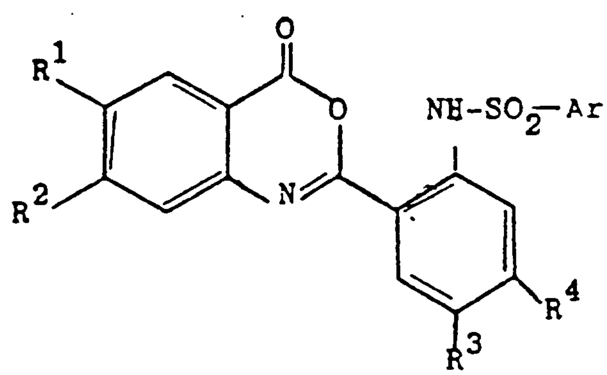

(I)

worin $R^1$, $R^2$, $R^3$ und $R^4$ unabhängig H, Z oder Z-substituiertes Phenyl sind; und Ar optional Z-substituiertes Phenyl oder Heterozyklyl; in dem die Z unabhängig ausgewählt sind aus $C_{1-4}$ Alkyl, OH, $C_{1-4}$ Alkoxy, Halogen, $NO_2$, $NH_2$, $C_{1-4}$ Alkylamino, di($C_{1-4}$ Alkyl)Amino und Phenyl; vorausgesetzt, daß $R^1$, $R^2$, $R^3$ und $R^4$ nicht alle H sind, wenn Ar Aklylphenyl oder Alkoxyphenyl ist.

2. Farbe nach Anspruch 1, worin $R^1$, $R^2$, $R^3$ und $R^4$ unabhängig ausgewählt sind aus H und Cl oder einem anderen Halogen.

3. Farbe nach irgendeinem vorhergehenden Anspruch, worin $R^1 = R^3$ und $R^2 = R^4$.

4. Farbe nach einem der vorangehenden Ansprüche, worin einer aus $R^1$ und $R^2$ H ist und einer aus $R^3$ und $R^4$ H ist.

5. Farbe nach Anspruch 1, worin $R^1$, $R^2$, $R^3$ und $R^4$ jeweils H sind.

6. Farbe nach einem der vorhergehenden Ansprüche worin Ar ein Z-substituiertes Phenyl außer Alkylphenyl oder Nitrophenyl ist.

7. Farbe nach Anspruch 6, worin Ar Biphenylyl ist.

8. Farbe nach einem der vorhergehenden Ansprüche, worin Ar 4-(Z-substituiertes)Phenyl ist.

9. Farbe nach einem der Ansprüche 1 bis 5, worin Ar 2 oder 3-Thienyl oder 2,3 oder 4-Pyridyl ist.

**10.** Sicherheitsdokument, das eine Verbindung gemäß einem der vorhergehenden Ansprüche trägt.

**11.** Verbindung nach Formel I, wie sie in einem der Ansprüche 1 bis 10 definiert ist, vorausgesetzt daß, wenn $R^1$, $R^2$, $R^3$ und $R^4$ ausgewählt sind aus H, Alkyl, Alkoxy und Halogen, Ar nicht Phenyl, Alkylphenyl oder Alkoxyphenyl ist, und daß die Verbindung nicht 2-[2-(4-Nitrophenylsulfonylamino)-Phenyl]-4H-3,1-benzoxazin-4-on oder 2-[2-(4-Methylphenylsulfonylamino)-4-Nitrophenyl]-4H-3,1-Benzoxazin-4-on ist.

**12.** Verbindung nach Anspruch 11, die 2-[2-(4-Biphenylylsulfonylamino)Phenyl]-4H-3,1-Benzoxazin-4-on ist.

**13.** Verbindung nach Anspruch 11, die 2-[2-(4-Chlorphenylsulfonylamino)Phenyl]-4H-3,1-Benzoxazin-4-on ist.

**14.** Verbindung nach Anspruch 11, die 2-[2-(4-Methylphenylsulfonylamino)-5-Chlorphenyl]-6-Chlor-4H-3,1-Benzoxazin-4-on ist.

**15.** Verbindung nach Anspruch 11, die 2-[2-(4-Chlorphenylsulfonylamino)-5-Chlorphenyl]-6-Chlor-4H-3,1-Benzoxazin-4-on ist.

**16.** Verfahren zur Herstellung einer Verbindung der in Anspruch 1 gezeigten Formel, worin $R^1$ und $R^3$ oder $R^2$ und $R^4$ Halogene sind, das umfaßt Reagieren einer entsprechenden optional-substituierten Anthranilsäure der Formel III

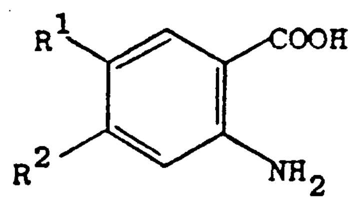

(III)

mit einem carbozyklischen oder heterozyklischen Arylsulfonylchlorid.

**17.** Verfahren zum Herstellen einer Verbindung der in Anspruch 1 gezeigten Formel, wobei $R^1$, $R^2$, $R^3$, $R^4$ und Ar optional wie in Anspruch 1 sind, das umfaßt (1) Reagieren einer Anthranilsäure der Formel III

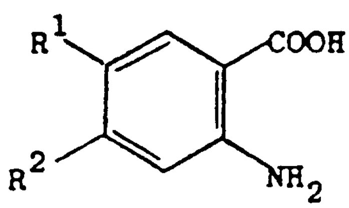

(III)

mit $SOCl_2$; und (2) Reagieren des sich ergebenden Zwischenprodukts von Formel IV

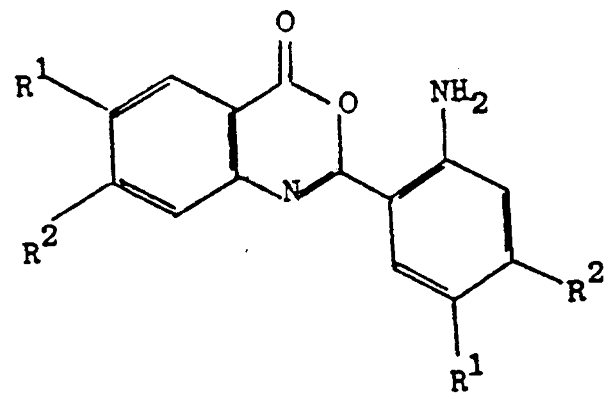

(IV)

mit einem carbozyklischen oder heterozyklischen Arylsulfonylchlorid der Formel Ar-$SO_2$-Cl.

**Revendications**

1. Encre fluorescente comprenant un composé de formule (I)

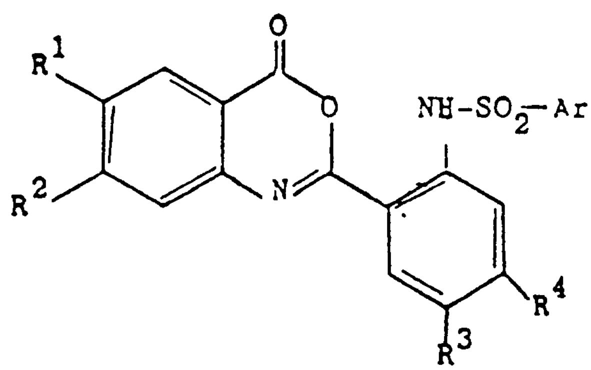

(I)

dans laquelle $R^1$, $R^2$, $R^3$ et $R^4$ sont indépendamment H, Z ou un groupe phényle substitué par Z; et Ar est un groupe phényle ou un reste hétérocyclique facultativement substitué par Z ; et dans laquelle chaque reste Z est choisi parmi un halogène, un groupe alkyle en $C_1$-$C_4$, un groupe OH, un groupe alcoxy en $C_1$-$C_4$, un groupe $NO_2$, un groupe $NH_2$, un groupe alkylamino en $C_1$-$C_4$, un groupe di(alkyl en $C_1$-$C_4$)amino et un groupe phényle; à la condition que $R^1$, $R^2$, $R^3$ et $R^4$ ne soient pas tous H lorsque Ar est un groupe alkylphényle ou alcoxyphényle.

2. Encre selon la revendication 1, dans laquelle $R^1$, $R^2$, $R^3$ et $R^4$ sont choisis chacun parmi H et Cl ou un autre halogène.

3. Encre selon l'une ou l'autre des revendications précédentes, dans laquelle $R^1 = R^3$ et $R^2 = R^4$.

4. Encre selon l'une quelconque des revendications précédentes, dans laquelle l'un des restes $R^1$ et $R^2$ est H et l'un des restes $R^3$ et $R^4$ est H.

5. Encre selon la revendication 1, dans laquelle $R^1$, $R^2$, $R^3$ et $R^4$ sont chacun H.

6. Encre selon l'une quelconque des revendications précédentes, dans laquelle Ar est un groupe phényle substitué par Z autre qu'un groupe alkylphényle ou nitrophényle.

7. Encre selon la revendication 6, dans laquelle Ar est un groupe biphénylyle.

8. Encre selon l'une quelconque des revendications précédentes, dans laquelle Ar est un groupe 4-Z-phényle.

9. Encre selon l'une quelconque des revendications 1 à 5, dans laquelle Ar est un groupe 2 ou 3-thiényle ou 2,3 ou 4-pyridyle.

10. Document de sécurité portant un composé selon l'une quelconque des revendications précédentes.

11. Composé de formule I définie dans l'une quelconque des revendications 1 à 10, pourvu que, lorsque $R^1$, $R^2$, $R^3$ et $R^4$ sont choisis parmi H, un groupe alkyle et un groupe alcoxy, Ar ne soit pas un groupe phényle, alkylphényle ou alcoxyphényle et que le composé ne soit pas la 2-[2-(4-nitrophénylsulfonylamino)-phényl]-4H-3,1-benzoxazine-4-one ou la 2-[2-(4-méthylphénylsulfonylamino)-4-nitrophényl]-4H-3,1-benzoxazine-4-one.

12. Composé selon la revendication 11, qui est la 2-[2-4-biphénylsulfonylamino)phényl]-4H-3,1-benzoxazine-4-one.

13. Composé selon la revendication 11, qui est la 2-[2-(4-chlorophénylsulfonylamino)phényl]-4H-3,1-benzoxazine-4-one.

14. Composé selon la revendication 11, qui est la 2-[2-(4-méthylphénylsulfonylamino)-5-chlorophényl]-6-chloro-4H-3,1-benzoxazine-4-one.

15. Composé selon la revendication 11, qui est la 2-[2-(4-chlorophénylsulfonylamino)-5-chlorophényl]-6-chloro-4H-3,1-benzoxazine-4-one.

16. Procédé pour préparer un composé de formule représentée à la revendication 1, dans laquelle $R^1$ et $R^3$ ou $R^2$ et $R^4$ sont des halogènes, qui comprend la réaction d'un acide anthranilique facultativement substitué correspondant de formule III.

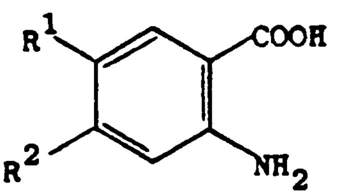

(III)

avec un chlorure d'arylsulfonyle carbocyclique ou hétérocyclique.

17. Procédé pour préparer un composé de formule représenté à la revendication 1, $R^1$, $R^2$, $R^3$, $R^4$ et Ar étant facultativement définis comme à la revendication 1, qui comprend (1) la réaction d'un acide anthranilique de formule III

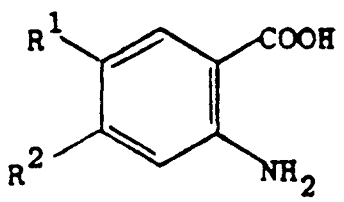

(III)

avec $SOCl_2$ ; et (2) la réaction de l'intermédiaire résultant de formule IV

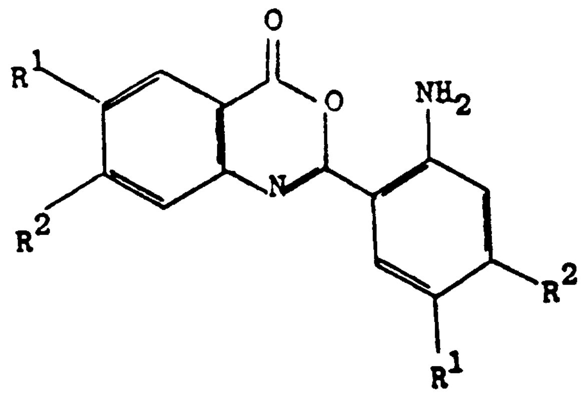

(IV)

avec un chlorure d'arylsulfonyle carbocyclique ou hétérocyclique de formule $Ar\text{-}SO_2Cl$.